# EUROPÄISCHE PATENTSCHRIFT

(11) **EP 0 802 903 B1**
(45) Veröffentlichungstag und Bekanntmachung des Hinweises auf die Patenterteilung: **09.09.1998**
(21) Anmeldenummer: 96900557.8
(22) Anmeldetag: 04.01.1996
(51) Int. Cl.: C07D 213/61, A01N 43/40

(54) **SUBSTITUIERTE 2-PHENYLPYRIDINE ALS HERBIZIDE**
SUBSTITUTED 2-PHENYLPYRIDINES AS HERBICIDES
2-PHENYLPYRIDINES SUBSTITUEES UTILISEES COMME HERBICIDES

(30) Priorität: 13.01.1995 DE 19500911
(43) Veröffentlichungstag der Anmeldung: 29.10.1997
(73) Patentinhaber: BASF AKTIENGESELLSCHAFT, 67056 Ludwigshafen (DE)
(72) Erfinder: SCHÄFER, Peter, D-67308 Ottersheim (DE); HAMPRECHT, Gerhard, D-69469 Weinheim (DE); HEISTRACHER, Elisabeth, D-67061 Ludwigshafen (DE); KLINTZ, Ralf, D-67269 Grünstadt (DE); KÖNIG, Hartmann, D-69115 Heidelberg (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: EP9600008
(87) Internationale Veröffentlichungsnummer: WO9621646

(56) Entgegenhaltungen:
- EP-A- 0 463 492
- DE-A- 4 323 916
- CHEMICAL ABSTRACTS, vol. 114, no. 11, 18.März 1991 Columbus, Ohio, US; abstract no. 96724k, N.P. MEL'NIKOVA ET AL. 'Photodegradation of the herbicide Zellek.' Seite 243; & IZVESTIYA TIMIRYAZEVSKOI SEL'SKOKHOZYAISTVENNOI AKADEMII, Nr. 3, 1990 MOSCOW, Seiten 155-160,

## Beschreibung

Die vorliegende Erfindung betrifft neue substituierte 2-Phenylpyridine der Formel I 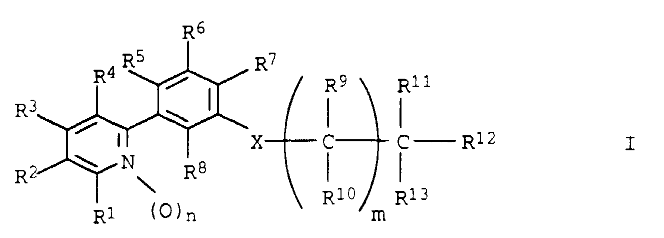 in der die Variablen folgende Bedeutung haben:
- n: 0 oder 1;
- R¹, R³, R⁴: unabhängig voneinander
Wasserstoff, Halogen, C₁-C₄-Alkyl, C₁-C₄-Halogenalkyl, Hydroxy, C₁-C₄-Alkoxy, C₁-C₄-Halogenalkoxy, Nitro, Amino, C₁-C₄-Alkylamino, Di-(C₁-C₄-alkyl)-amino, Mercapto, C₁-C₄-Alkylthio, C₁-C₄-Halogenalkylthio, Cyano, Carboxyl, Aminocarbonyl, (C₁-C₄-Alkyl-amino)carbonyl oder Di-(C₁-C₄-alkyl)-aminocarbonyl;
- R²: Wasserstoff, Halogen, C₁-C₄-Alkyl, C₁-C₄-Halogenalkyl, Cyano, Nitro, Amino, Hydroxy, C₁-C₄-Halogenalkoxy, Mercapto, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
- R⁵: Wasserstoff, Halogen oder Cyano;
- R⁶ und R⁸: unabhängig voneinander Wasserstoff oder Halogen;
- R⁷: Wasserstoff, Cyano, Nitro, Hydroxy, Trifluormethylsulfonyloxy, Halogen, C₁-C₄-Alkyl, C₁-C₄-Halogenalkyl, C₁-C₄-Alkoxy oder C₁-C₄-Halogenalkoxy;
- X: Sauerstoff oder Schwefel,
- R⁹, R¹⁰: unabhängig voneinander Wasserstoff oder C₁-C₄-Alkyl;
- m: 1, 2, 3 oder 4;
- R¹¹: C₁-C₆-Alkyl, C₃-C₆-Cycloalkyl, C₁-C₄-Halogenalkyl mit ein bis fünf Halogenatomen, C₂-C₄-Alkenyl, C₂-C₄-Halogenalkenyl mit ein bis drei Halogenatomen, (C₁-C₄-Alkoxy)carbonyl, (C₁-C₄-Alkoxy)carbonyl-(C₁-C₄-alkyl)-, unsubstituiertes oder ein bis dreifach substituiertes Phenyl, unsubstituiertes oder ein oder zweifach substituiertes Thienyl, Furanyl, Tetrahydrofuranyl, Pyrrolyl, Oxazolyl, Isoxazolyl, Thiazolyl, Isothiazolyl, Pyridyl oder Pyrimidyl, wobei die Substituenten jeweils ausgewählt sind aus der Gruppe bestehend aus Nitro, Cyano, Halogen, C₁-C₄-Alkyl und C₁-C₄-Alkoxy;
- R¹², R¹³: unabhängig voneinander C₁-C₄-Alkoxy oder C₁-C₄-Alkylthio oder zusammen mit dem gemeinsamen Kohlenstoffatom, an das sie gebunden sind, die Carbonylgruppe, eine Gruppe

〉C=N―O―R¹⁴

wobei R¹⁴ für Wasserstoff, C₁-C₄-Alkyl, C₂-C₆-Alkenyl, C₂-C₆-Halogenalkenyl mit ein bis drei Halogenatomen, (C₁-C₄-Alkoxy)carbonyl-(C₁-C₄-alkyl)- oder Benzyl steht, oder einen Heterocyclus 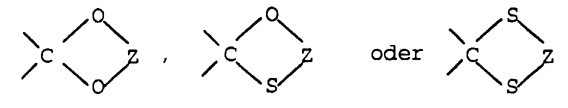 wobei Z für eine Ethylen- oder Trimethylenkette steht, bei der gewünschtenfalls ein bis vier Wasserstoffatome durch jeweils C₁-C₄-Alkyl oder (C₁-C₄-Alkoxy)carbonyl substituiert sein können,
sowie die landwirtschaftlich brauchbaren Salze der Verbindungen I, sofern diese existieren.

Außerdem betrifft die Erfindung
- die Verwendung der Verbindungen I als Herbizide und/oder zur Desikkation/Defoliation von Pflanzen,
- herbizide Mittel und Mittel zur Desikkation und/oder Defoliation von Pflanzen, welche die Verbindungen I als wirksame Substanzen enthalten,
- Verfahren zur Bekämpfung von unerwünschtem Pflanzenwuchs und zur Desikkation/Defoliation von Pflanzen mit den Verbindungen I sowie
- Verfahren zur Herstellung der Verbindungen I und von herbiziden Mitteln und Mitteln zur Desikkation/Defoliation von Pflanzen unter Verwendung der Verbindungen I.

In der älteren deutschen Anmeldung DE-A 43 23 916 Veröffentlichungsdatum 19.01.95 : Stand der Technik im Sinne des Artikels 54 Absatz 3 EPÜ. werden u.a. 2-Phenylpyridine vom Typ der Verbindungen I als Herbizide und als desikkant/defoliant wirksame Verbindungen beschrieben. Bei geeigneter Wahl der Substituenten ergeben sich u.a. Verbindungen der Formel II 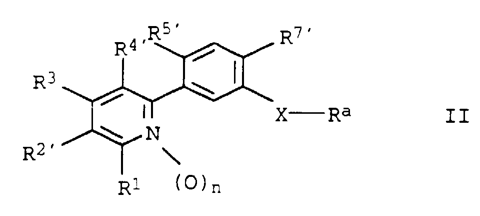 wobei
- R^{2'}: für Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
- R^{4'}: für Wasserstoff, Nitro, Amino, Cyano, Hydroxy, Mercapto, Hydroxycarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-(C₁-C₄-alkyl)-amino, C₁-C₄-Alkylthio, C₁-C₄-Halogenalkylthio oder (C₁-C₄-Alkoxy)carbonyl;
- R^{5'}: für Wasserstoff oder Halogen;
- R^{7'}: für Cyano, Nitro, Hydroxyl, Halogen oder Trifluormethyl;
- R^{a}: für Wasserstoff oder verschiedene organische Reste stehen.

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen.

Die Aufgabe erstreckt sich auch auf die Bereitstellung neuer desikkant/defoliant wirksamer Verbindungen.

Demgemäß wurden die eingangs definierten substituierten 2-Phenylpyridine der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die substituierten 2-Phenylpyridine I können in Form ihrer landwirtschaftlich brauchbaren Salze vorliegen, wobei es auf die Art des Salzes in der Regel nicht ankommt. Im allgemeinen kommen die Salze von solchen Basen und diejenigen Säureadditionssalze in Betracht, bei denen die herbizide Wirkung im Vergleich zu der freien Verbindung I nicht negativ beeinträchtigt ist.

Als Salze eignen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis vier C₁-C₄-Alkyl-, Hydroxy-C₁-C₄-alkylsubstituenten und/oder einen Phenyl- oder Benzyl-substituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-(C₁-C₄-alkyl)sulfonium-salze, und Sulfoxoniumsalze wie vorzugsweise Tri-(C₁-C₄-alkyl)sulfoxoniumsalze.

Unter den Säureadditionssalzen sind in erster Linie die Hydrochloride und -bromide, Sulfate, Nitrate, Phosphate, Oxalate und die Dodecylbenzolsulfonate zu nennen.

Die bei der Definition der Substituenten R¹ bis R¹⁴ sowie der Kette Z verwendeten Bezeichnungen Alkyl, Halogenalkyl, Alkoxy, Halogenalkoxy, Alkylthio, Halogenalkylthio, Alkylamino, Dialkylamino, Alkylaminocarbonyl, Dialkylaminocarbonyl, Alkylsulfinyl, Halogenalkylsulfinyl, Alkylsulfonyl, Halogenalkylsulfonyl, Alkenyl, Halogenalkenyl, Alkoxycarbonyl, Alkoxycarbonylalkyl und Cycloalkyl stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Alkenyl- und Halogenalkenyl-Teile können geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom oder Iod;
- C₁-C₄-Alkyl sowie der Alkyl-Teil von (C₁-C₄-Alkoxy)carbonyl-(C₁-C₄-alkyl)- für: Methyl, Ethyl, n-Propyl, 1-Methylethyl, n-Butyl, 1-Methylpropyl, 2-Methylpropyl oder 1,1-Dimethylethyl;
- C₁-C₆-Alkyl für: C₁-C₄-Alkyl wie vorstehend genannt, sowie für n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder l-Ethyl-2-methylpropyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₄-Alkoxy für: Methoxy, Ethoxy, n-Propoxy, 1-Methylethoxy, n-Butoxy, 1-Methylpropoxy, 2-Methylpropoxy oder 1,1-Dimethylethoxy;
- C₁-C₄-Halogenalkoxy für: C₁-C₄-Alkoxy wie vorstehend genannt, das partiell oder vollständig durch Fluor, Chlor, Brom und/ oder Iod substituiert ist, also z.B. Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorporpoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlor-propoxy, 2,2,3,3,3-Pentafluorpropoxy, Heptafluorporpoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- (C₁-C₄-Alkoxy)carbonyl sowie der Alkoxycarbonyl-Teil von (C₁-C₄-Alkoxy)carbonyl- (C₁-C₄-alkyl)- für: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl oder 1,1-Dimethylethoxycarbonyl;
- C₁-C₄-Alkylthio für: Methylthio, Ethylthio, n-Propylthio, 1-Methylethylthio, n-Butylthio, 1-Methylpropylthio, 2-Methylpropylthio oder 1,1-Dimethylethylthio;
- C₁-C₄-Halogenalkylthio für: C₁-C₄-Alkylthio wie vorstehend genannt, das partiell oder vollständig durch Fluor, Chlor, Brom und/oder Iod substituiert ist, also z.B. Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2,-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2,-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₄-Alkylsulfinyl für: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl oder 1,1-Dimethylethylsulfinyl;
- C₁-C₄-Halogenalkylsulfinyl für: C₁-C₄-Alkylsulfinyl wie vorstehend genannt, das partiell oder vollständig durch Fluor, Chlor, Brom und/oder Iod substituiert ist, also z.B. Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2,-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2,-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl) -2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl oder Nonafluorbutylsulfinyl;
- C₁-C₄-Alkylsulfonyl für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl;
- C₁-C₄-Halogenalkylsulfonyl für: C₁-C₄-Alkylsulfonyl wie vorstehend genannt, das partiell oder vollständig durch Fluor, Chlor, Brom und/oder Iod substituiert ist, also z.B. Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2,-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2,-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl;
- C₁-C₄-Alkylamino sowie der Alkylamino-Teil von (C₁-C₄-Alkylamino)carbonyl für: Methylamino, Ethylamino, n-Propylamino, 1-Methylethylamino, n-Butylamino, 1-Methyl-propylamino, 2-Methylpropylamino oder 1,1-Dimethylethylamino;
- Di-(C₁-C₄-alkyl)-amino sowie der Dialkylamino-Teil von Di-(C₁-C₄-alkyl)-aminocarbonyl für: z. B. N,N-Dimethylamino, N,N-Diethylamino, N,N-Diisopropylamino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)-amino, N-Methyl-N-(2-methylproyl)amino, N-(1,1-Dimethylethyl)-N-methylamino, N-Ethyl-N-propylamino, N-Ethyl-N-(1-methylethyl) amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methyletyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1,-Dimethylethyl)-N-(1-methylethyl)amino, N-Butyl-N-(1-methylpropyl)amino-, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino;
- C₃-C₆-Cycloalkyl für: Cyclopropyl, Cyclobutyl, Cyclopentyl oder Cyclohexyl;
- C₂-C₄-Alkenyl für: Ethinyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methylprop-2-en-1-yl oder 2-Methyl-prop-2-en-1-yl;
- C₂-C₆-Alkenyl für: C₂-C₄-Alkyl wie vorstehend genannt, sowie z.B. für n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₄-Halogenalkenyl für: C₂-C₄-Alkenyl wie vorstehend genannt, das partiell oder vollständig durch Fluor, Chlor, Brom und/oder Iod substituiert ist, also z.B. 2-Chlorallyl, 3-Chlorallyl oder 3,3-Dichlorallyl;
- C₂-C₆-Halogenalkenyl für: C₂-C₆-Alkenyl wie vorstehend genannt, das partiell oder vollständig durch Fluor, Chlor, Brom und/oder Iod substituiert ist, also z.B. 1-Chlorvinyl, 2-Chlorvinyl, 2,2-Dichlorvinyl, 1,2,3-Trichlorvinyl, 2-Chlorallyl, 3-Chlorallyl oder 3,3-Dichlorallyl.

Der Phenylring und die heterocyclischen Ringe in der Definition von R¹¹ sind vorzugsweise unsubstituiert oder tragen einen Halogen-, Methyl- oder Methoxy-Substituenten.

Im Hinblick auf die Verwendung der erfindungsgemäßen Verbindungen der Formel I als Herbizide und/oder als defoliant/desiccant wirksame Verbindungen haben die Variablen vorzugsweise folgende Bedeutungen, und war jeweils für sich allein oder in Kombination:
- n: Null;
- R¹, R³, R⁴: unabhängig voneinander Wasserstoff oder Halogen;
- R²: Halogen oder C₁-C₄-Halogenalkyl mit ein bis fünf Halogenatomen;
- R⁵: Wasserstoff oder Halogen, insbesondere Fluor;
- R⁶, R⁸: Wasserstoff;
- R⁷: Cyano oder Halogen, insbesondere Cyano oder Chlor;
- X: Sauerstoff;
- R⁹, R¹⁰: unabhängig voneinander Wasserstoff oder C₁-C₄-Alkyl;
- m: eins oder zwei;
- R¹¹: C₁-C₆-Alkyl oder C₃-C₆-Cycloalkyl;
- R¹² und R¹³: zusammen mit dem gemeinsamen Kohlenstoffatom, an das sie gebunden sind, die Carbonylgruppe.

Besonders bevorzugt sind die in der folgenden Tabelle 1 aufgeführten Verbindungen Ia (≙ I mit n = 0; R¹, R³, R⁶, R⁸ und R¹⁰ = Wasserstoff; R² = CF₃; R⁴ = Chlor; R¹¹ = Methyl; R¹² und R¹³ zusammen mit dem gemeinsamen C-Atom = Carbonyl):

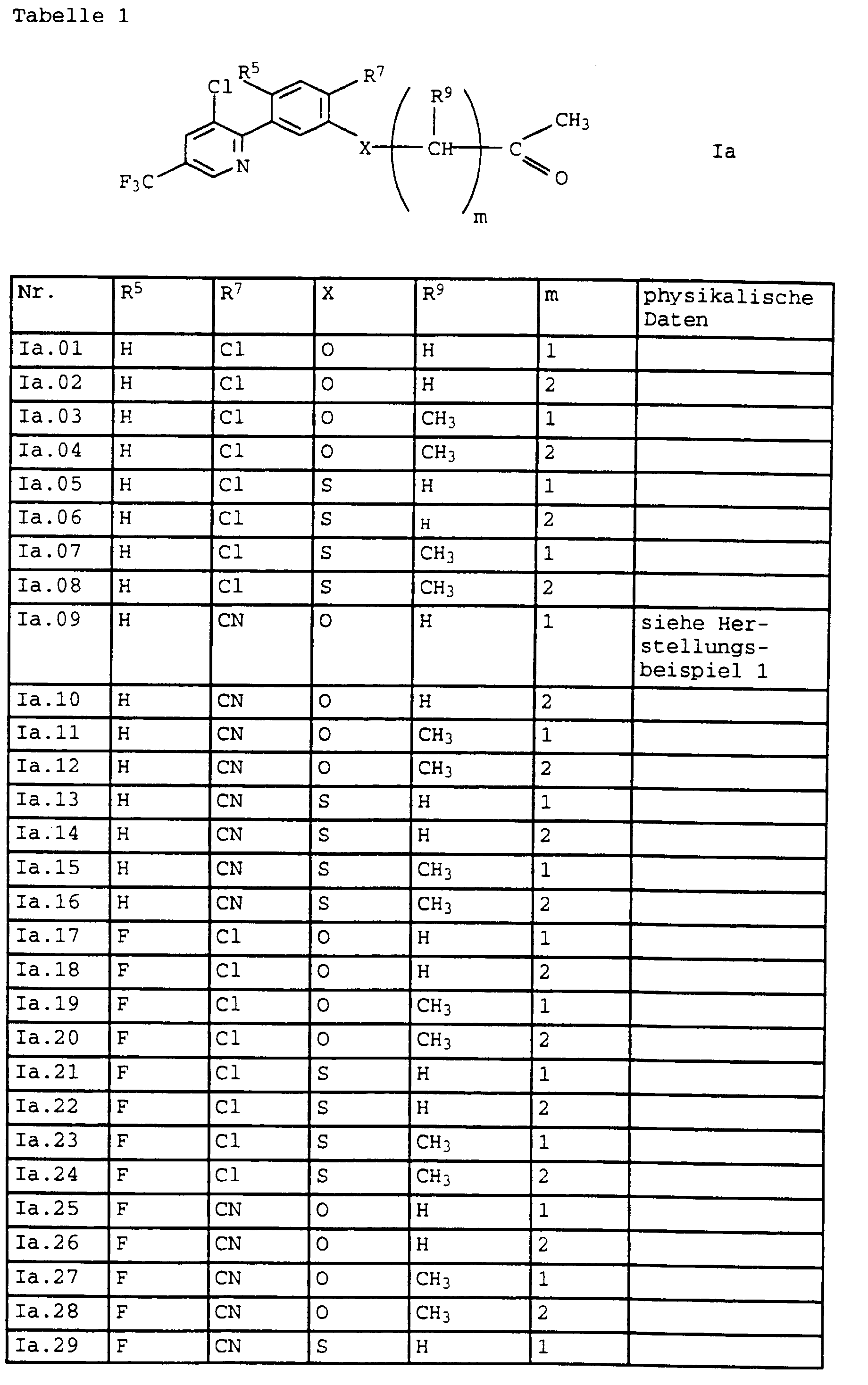

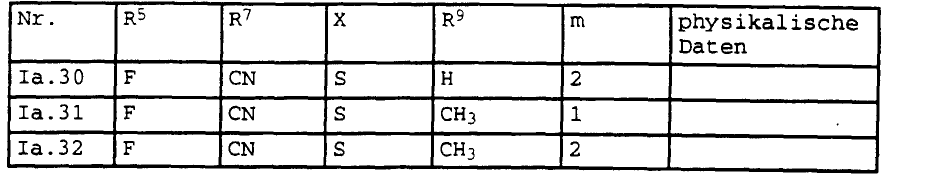

Des weiteren sind die folgenden substituierten 2-Phenylpyridine der Formel I besonders bevorzugt:
- die Verbindungen Ib.01 - Ib.32, die sich von den entsprechenden Verbindungen Ia.01 - Ia.32 lediglich dadurch unterscheiden, daß R¹¹ Ethyl bedeutet: 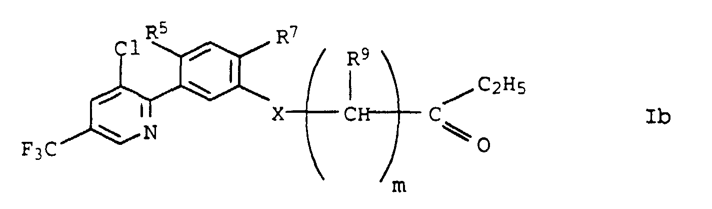
- die Verbindungen Ic.01 - Ic.32, die sich von den entsprechenden Verbindungen Ia.01 - Ia.32 lediglich dadurch unterscheiden, daß R¹¹ n-Propyl bedeutet: 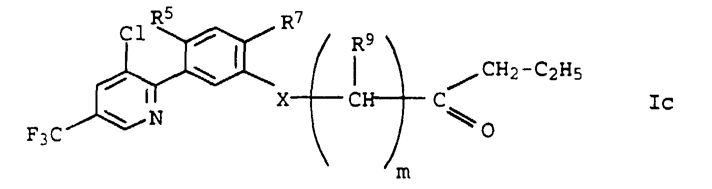
- die Verbindungen Id.01 - Id.32, die sich von den entsprechenden Verbindungen Ia.01 - Ia.32 lediglich dadurch unterscheiden, daß R¹¹ Isopropyl bedeutet: 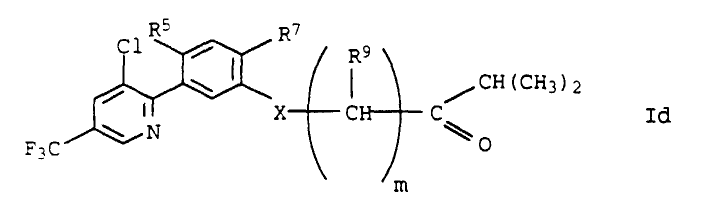
- die Verbindungen Ie.01 - Ie.32, die sich von den entsprechenden Verbindungen Ia.01 - Ia.32 lediglich dadurch unterscheiden, daß R¹¹ n-Butyl bedeutet: 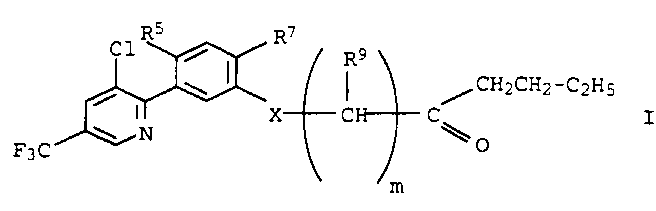
- die Verbindungen If.01 - If.32, die sich von den entsprechenden Verbindungen Ia.01 - Ia.32 lediglich dadurch unterscheiden, daß R¹¹ 2-Methylpropyl bedeutet: 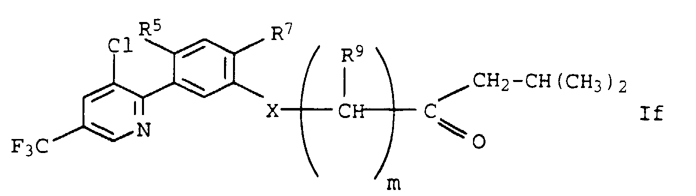
- die Verbindungen Ig.01 - Ig.32, die sich von den entsprechenden Verbindungen Ia.01 - Ia.32 lediglich dadurch unterscheiden, daß R¹¹ tert.-Butyl bedeutet: 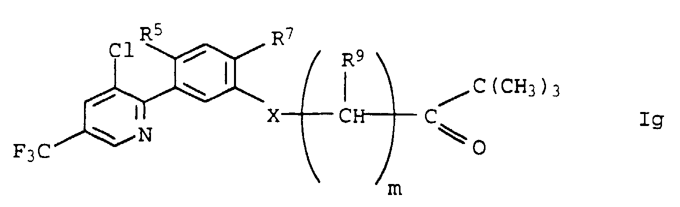
- die Verbindungen Ih.01 - Ih.32, die sich von den entsprechenden Verbindungen Ia.01 - Ia.32 lediglich dadurch unterscheiden, daß R¹¹ Cyclopropyl bedeutet: 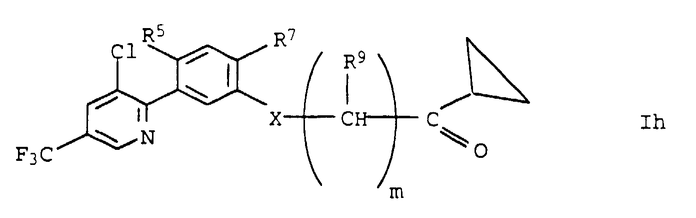
- die Verbindungen Ii.01 - Ii.32, die sich von den entsprechenden Verbindungen Ia.01 - Ia.32 lediglich dadurch unterscheiden, daß R¹¹ Methoxycarbonylmethyl bedeutet: 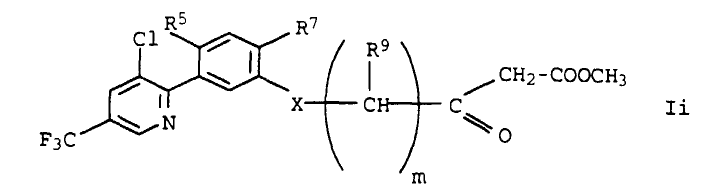
- die Verbindungen Ik.01 - Ik.32, die sich von den entsprechenden Verbindungen Ia.01 - Ia.32 lediglich dadurch unterscheiden, daß R¹¹ Ethoxycarbonylmethyl bedeutet:

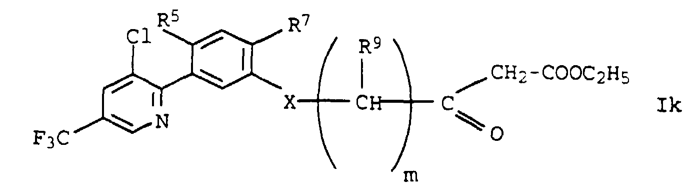

Außerdem sind die in der folgenden Tabelle 2 aufgeführten Verbindungen Il (≙ I mit n = 0; R¹, R³, R⁶, R⁸ und R¹⁰ = Wasserstoff; R² = CF₃; R⁴ = Chlor; m = 1; R¹¹ = Methyl; R¹², R¹³ = Methoxy) besonders bevorzugt:

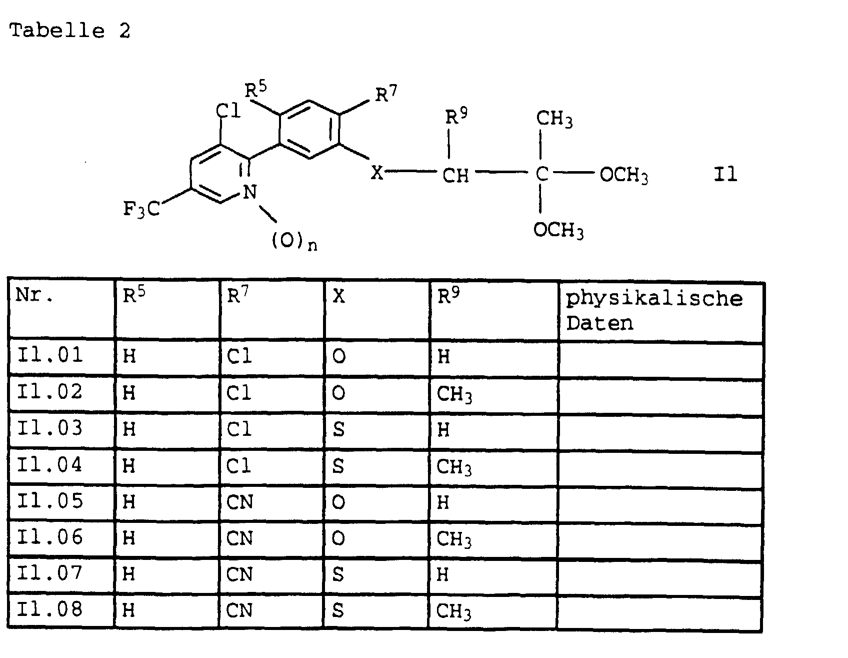

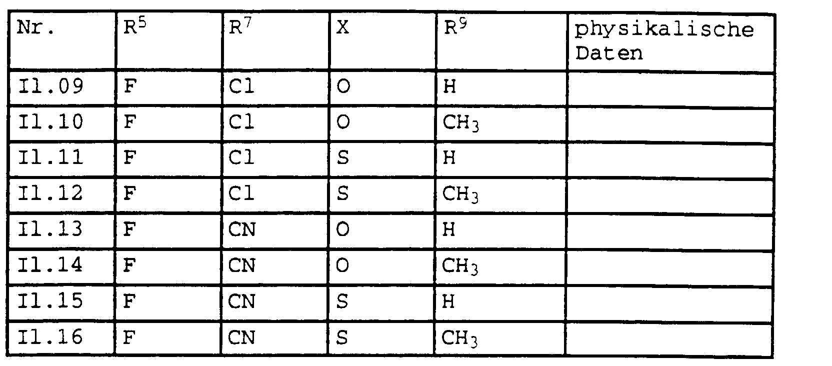

Schließlich sind noch die folgenden substituierten 2-Phenylpyridine I besonders bevorzugt:
- die Verbindungen Im.01 - Im.16, die sich von den entsprechenden Verbindungen Il.01 - Il.16 lediglich dadurch unterscheiden, daß R¹² und R¹³ Ethoxy bedeuten: 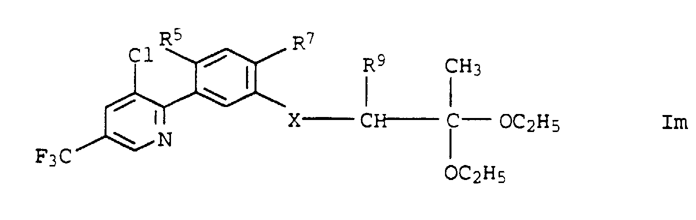
- die Verbindungen In.01 - In.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, einen 1,3-Dioxolanring bilden: 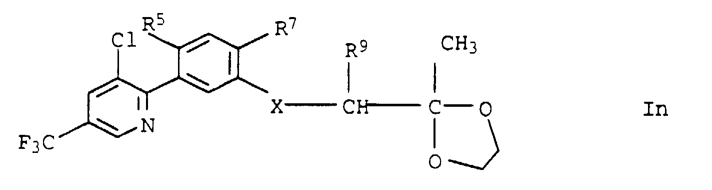
- die Verbindungen Io.01 - Io.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, einen 1,3-Dioxanring bilden: 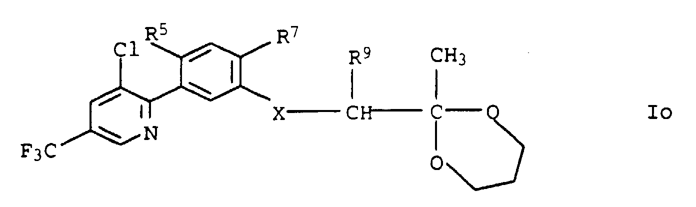
- die Verbindungen Ip.01 - Ip.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, einen 1,3-Dithiolanring bilden: 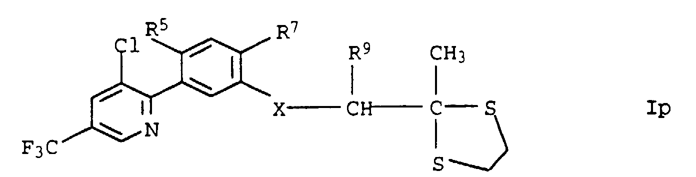
- die Verbindungen Iq.01 - Iq.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, einen 1,3-Dithianring bilden: 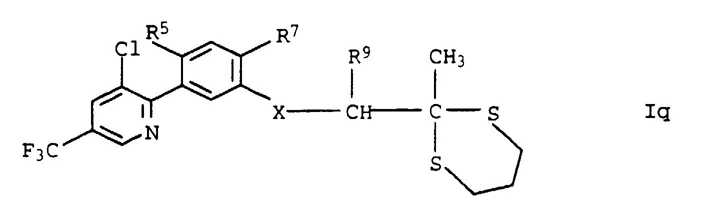
- die Verbindungen Ir.01 - Ir.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, eine Gruppe

   〉C=N―OH

   bilden: 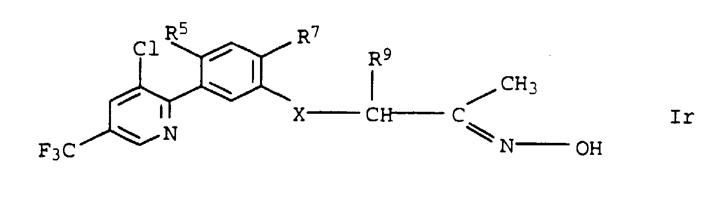
- die Verbindungen Is.01 - Is.16, die sich von den entsprechenden Verbindungen Il.01 - Il.16 lediglich dadurch unterscheiden, daß R¹² und R¹³ zusammen mit dem gemeinsamen C-Atom, an das sie gebunden sind, eine Gruppe

   〉C=N―OCH₃

   bilden: 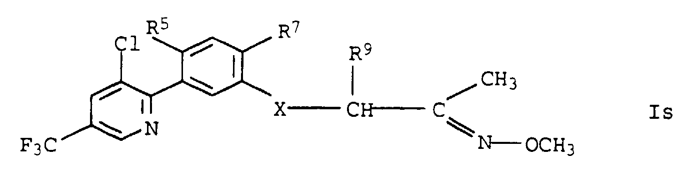
- die Verbindungen It.01 - It.16, die sich von den entsprechenden Verbindungen Il.01 - Il.32 lediglich dadurch unterscheiden, daß R¹² und R¹³ zusammen mit dem gemeinsamen C-Atom, an das sie gebunden sind, eine Gruppe

   〉C=N―OC₂H₅

   bilden:

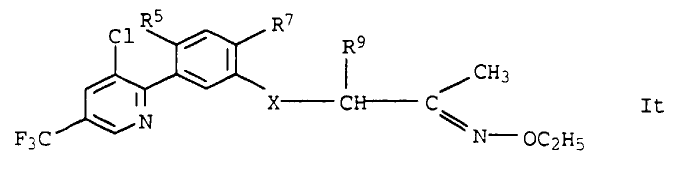

Die substituierten 2-Phenylpyridine der Formel I sind auf verschiedene Weise erhältlich, beispielsweise nach einem der folgenden Verfahren:

### Verfahren A

Alkylierung von 3-Pyridylphenolen oder 3-Pyridylthiophenolen III mit Alkylierungsmitteln IV in Gegenwart einer Base

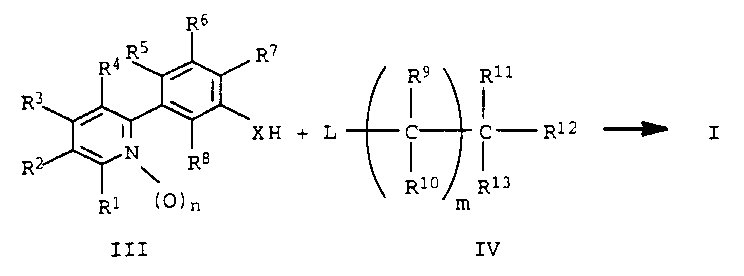

L steht für Chlor, Brom, Jod, Methylsulfonyloxy, Trifluormethylsulfonyloxy, Phenylsulfonyloxy oder p-Tolylsulfonyloxy.

In der Regel arbeitet man in einem inerten Lösungs- oder Verdünnungsmittel, das vorzugsweise aprotisch ist, also z.B. in N,N-Di-methylformamid, Dimethylsulfoxid, Aceton, N-Methylpyrrolidon, Acetonitril oder in einem Ether wie Diethylether, Tetrahydrofuran und 1,4-Dioxan.

Brauchbare Basen sind beispielsweise Alkalimetallcarbonate und -hydrogencarbonate wie Natriumhydrogencarbonat, Kaliumhydrogencarbonat, Natriumcarbonat und Kaliumcarbonat, Alkalimetallalkoholate wie Natriummethanolat und Kalium-tert.-butanolat, Alkalimetallhydroxide wie Natriumhydroxid, und Alkalimetallhydride wie Natriumhydrid.

Weitere Angaben zur Durchführung derartiger Alkylierungsreaktionen sind z.B. in der folgenden Literatur zu finden:
- C.D. Hurd und P. Perletz, J. Am. Chem. Soc. 68, 38 (1946);
- W.M. Best und D. Wege, Aust. J. Chem. 39, 647 (1986);
- D. Salunkhe et al., Acta Chim. Hung. 124, 411 (1987);
- D.D. Weller et al., J. Org. Chem. 48, 3061 (1983);
- J.E. Banfield et al., J. Chem. Soc., 4791 (1956);
- P.S. Kukolja et al., J. Med. Chem. 28, 1886 (1985);
- K. Goerlitzer et al., Arch. Pharm. 313, 27 (1980);
- A. Shafiee et al., J. Het. Chem. 19, 1305 (1982);
- E. Campaigne und S. Kim Chung, J. Het. Chem. 20, 1697 (1983);
- S. Apparao und R.R. Schmidt, Synthesis 1987, 896.

### Verfahren B

Umsetzung von substituierten 2-Phenylpyridinen I, bei denen R¹² und R¹³ zusammen mit dem gemeinsamen Kohlenstoffatom, an das sie gebunden sind, die Carbonylgruppe bedeuten, entweder mit Hydroxylamin, wobei das Verfahrensprodukt anschließend alkyliert werden kann, oder mit einem Alkoxyamin H₂N-O-R¹⁴:

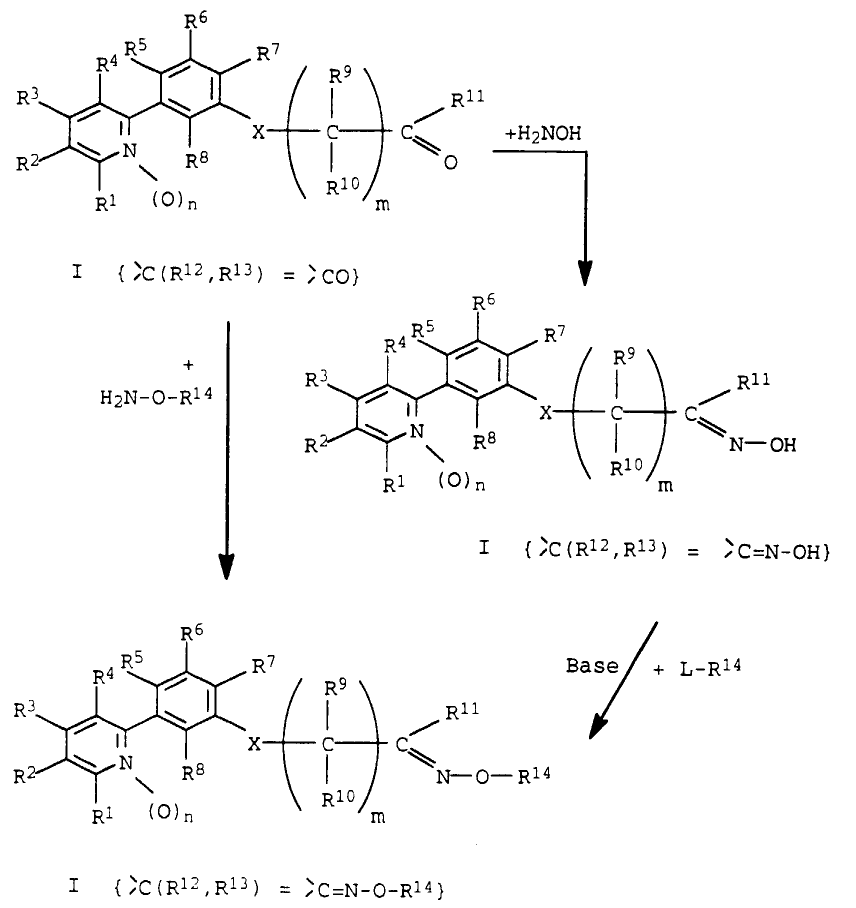

### Definition von L siehe Verfahren A.

Die Verfahrensbedingungen für derartige Umsetzungen von Ketonen mit Hydroxylamin(derivaten) sind beispielsweise der folgenden Literatur zu entnehmen:
- N. Bodor et al., J. Med. Chem. 31, 100 (1988);
- N. Latif et al., Indian J. Chem. B19, No. 4, 301-304 (1980);
- M. Watanabe et al., Chem. And Pharm. Bull. 32, 3551 (1984);
- P.H.H. Hermkens et al., Tetrahedron 46, 833 (1990);
- E.V. Dehmlow et al., Chem. Ber. 119, 2956 (1986);
- R. Plate et al., J. Chem. Soc. Perkin Trans. 1, 2478-2480 (1987);
- Y. Tsuda et al., Heterocycles 27, 63 (1988);
- J. Koyama et al., Heterocycles 29, 1649 (1989).

### Verfahren C

Umsetzung von substituierten 2-Phenylpyridinen I, bei denen R¹² und R¹³ zusammen mit dem gemeinsamen Kohlenstoffatom, an das sie gebunden sind, die Carbonylgruppe bedeuten in Gegenwart einer Säure,
entweder mit zweiwertigen Alkoholen oder Thioalkoholen oder mit einwertigen Alkoholen oder Thioalkoholen oder mit Ortho(thio)estern. Die Verfahrensprodukte I mit R¹², R¹³ = Alkoxy oder Alkylthio können gewünschtenfalls in Gegenwart einer Säure umacetalisiert werden:

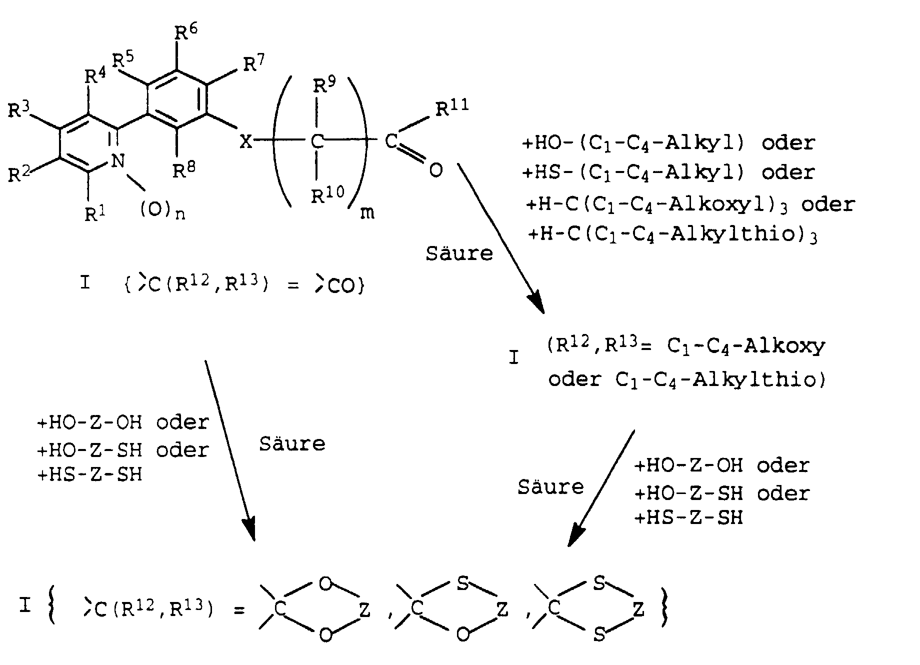

Weitere Angaben zur Durchführung derartiger Ketalbildungsreaktionen sind in der folgenden Literatur zu finden, auf die beispielhaft verwiesen wird:
- D.J. Collins und J.J. Hobbs, Chem. Ind., 1063 (1964);
- C.E. Ballov, Biochem. Prep., 45 (1960);
- C.L. Stevens und A.E. Sherr, J. Org. Chem. 17, 1228 (1952);
- J.L. Reymond und P. Vogel, J. Chem. Soc. Chem. Commun. 16, 1070 (1990);
- K.C. Nicolaov et al., Angew. Chem. Int. Ed. 30, 299 (1991);
- V. Rosnati et al., Gazz. Chim. Ital. 94, 767 (1964);
- W.M. Best und D. Wege, Aust. J. Chem. 39, 647 (1986);
- F. Kido et al., J. Chem. Soc. Chem. Commun. 8, 590 (1986);
- M.T. Barros et al., Tetrahedron 44, 2283 (1988);
- L.L. Melhado und J.L. Brodsky, J. Org. Chem. 53, 3852 (1988).

### Verfahren D

Oxidation von substituierten 2-Phenylpyridinen der Formel I, bei denen n Null bedeutet, auf an sich bekannte Weise {vgl. z.B. A. Albini u. S. Pietra, Heterocyclic N-Oxides, CRC-Press Inc., Boca Raton, USA 1991; H.S. Mosher et al., Org. Synth. Coll. Vol. IV 1963, Seite 828; E.C. Taylor et al., Org. Synth. Coll. Vol. IV 1963, Seite 704; T.W. Bell et. al., Org. Synth. 69, 226 (1990)}:

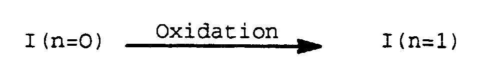

Unter den zur Oxidation des Pyridinrings üblichen Oxidationsmitteln sei beispielhaft auf Peressigsäure, Trifluorperessigsäure, Perbenzoesäure, m-Chlorperbenzoesäure, Monopermaleinsäure, Magnesiummonoperphthalat, Natriumperborat, Oxone® (enthält Peroxidisulfat), Perwolframsäure und Wasserstoffperoxid verwiesen.

Geeignete Lösungsmittel sind z.B. Wasser, Schwefelsäure, Carbonsäuren wie Essigsäure und Trifluoressigsäure sowie halogenierte Kohlenwasserstoffe wie Dichlormethan und Chloroform.

Normalerweise gelingt die Oxidation bei Temperaturen von 0 °C bis Siedetemperatur des Reaktionsgemisches.

Das Oxidationsmittel wird normalerweise in mindestens äuqimolaren Mengen, bezogen auf die Ausgangsverbindung, eingesetzt. Im allgemeinen hat sich ein Überschuß an Oxidaitonsmittel als besonders vorteilhaft erwiesen.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 2-Phenylpyridine der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Substituierte 2-Phenylpyridine I mit CH-aciden Substituenten lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium- und Phosphoniumsalze mittels Ammoniak, Phosphonium-, Sulfonium- oder Sulfoxonium-hydroxiden.

Verbindungen I, die eine endständige Aminogruppe tragen, können ferner Säureadditionssalze bilden. Geeignet sind allgemein die Salze von solchen Säuren, welche die herbizide oder desikkant/defoliante Wirkung von I ebenfalls nicht negativ beeinträchtigen, also z.B. die Hydrochloride und -bromide, Sulfate, Nitrate, Phosphate, Oxalate oder die Dodecylbenzolsulfonate.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwunschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vilgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (coffea canephora, coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die substituierten 2-Phenylpyridine I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen von 0,001 bis 98 Gew.%, vorzugsweise 0,01 bis 95 Gew.%, Wirkstoff. Die Wirkstoffe werden dabei in einer Reinheit von 90% bis 100%, vorzugsweise 95% bis 100%, (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.09 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.% des Wirkstoffs enthält.
II. 20 Gewichtsteile der Verbindung Nr. Ih.09 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.% des Wirkstoffs enthält.
III. 20 Gewichtsteile des Wirkstoffs Nr. Ia.09 werden in einer Mischung gelost, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.% des Wirkstoffs enthält.
IV. 20 Gewichtsteile des Wirkstoffs Nr. Ih.09 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.% des Wirkstoffs enthält.
V. 3 Gewichtsteile des Wirkstoffs Nr. Ia.09 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. Ih.09 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile Ölige Dispersion.
VII. 1 Gewichtsteil der Verbindung Nr. Ia.09 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. Ih.09 wird in einer Mischung gelöst, die aus 80 Gewichtsteilen Cyclohexanon und 20 Gewichtsteilen Emulphor EL¹⁾ besteht. Man erhält ein stabiles Emulsionskonzentrat.

^{**1)**} **ethoxyliertes Rizinusöl (caster-oil)**

Die Applikation der Wirkstoffe bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten 2-Phenylpyridine I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsaure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5, 6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### 3-Chlor-2-(4-cyano-3-(2-oxopropoxy)-phenyl)-5-trifluormethylpyridin (Nr. Ia.09)

1,5 g 3-Chlor-2-(4-cyano-3-hydroxyphenyl)-5-trifluormethylpyridin, 1,0 g Chloraceton, 1,5 g Kaliumcarbonat, 0,8 g Natriumjodid und 200 ml Aceton wurden 8 Stunden auf Rückflußtemperatur erhitzt. Anschließend engte man das Reaktionsgemisch ein, wonach der Rückstand in 20 ml Wasser verrührt wurde. Der unlöslich Anteil wurde abgetrennt und gut mit Wasser gewaschen. Danach verrührte man das Rohprodukt nacheinander mit n-Hexan und mit Ether. Nach Trocknen im Vakuumtrockenschrank erhielt man schließlich 0,4 g weißer Kristalle mit einem Schmelzpunkt von 146-147 °C. Ausbeute: 22 %.

### Vorstufe α:

### 3-Chlor-2-(4-cyano-3-methoxyphenyl)-5-trifluormethyl-pyridin

Zu einer Lösung von 49,0 g 3-Chlor-2-(4-cyano-3-nitrophenyl)-5-trifluormethylpyridin (bekannt aus der DE-A 43 23 916) in 500 ml wasserfreiem Methanol wurden 31 ml einer 30 gew.%igen Lösung von Natriummethylat in Methanol gegeben. Nach 6 Std. Erhitzen auf Rückflußtemperatur ließ man die Reaktionsmischung 16 Stunden bei 23°C stehen, wonach die mittlerweile entstandenen Kristalle abgetrennt, mit wenig Methanol gewaschen und schließlich im Vakuumtrockenschrank getrocknet wurden. Ausbeute: 33,3 g (71 %) weißer Kristalle mit einem Schmelzpunkt von 135-137°C.

### Vorstufe β:

### 3-Chlor-2-(4-cyano-3-hydroxyphenyl)-5-trifluormethylpyridin

5,0 g 3-Chlor-2-(4-cyano-3-methoxyphenyl) -5-trifluormethylpyridin und 5,5 g Pyridinhydrochlorid wurden zwei Stunden bei 200°C gerührt. Nach dem Abkühlen rührte man die Reaktionsmischung mit 100 ml Wasser gut durch. Anschließend wurde der Feststoffanteil abgetrennt und mittels Säulenchromatographie an Kieselgel (Cyclohexan/Methyl-tert.-butylether (2:1) als Elutionsmittel) gereinigt. Ausbeute: 3,4 g (71 %) weißer Kristalle mit einem Schmelzpunkt von 155-157°C.
¹H-NMR (250 MHz, in d⁶-Dimethylsulfoxid): δ [ppm] = 7.28(d,1H), 7.48(s,1H), 7.79(d,1H), 8.62(s,1H), 9.06(s,1H), 11.49(s.1H).

### Beispiel 2

### 3-Chlor-2- (4-cyano-3-cyclopropylcarbonylmethoxyphenyl)-5-trifluormethylpyridin (Nr. Ih.09)

Eine Mischung aus 1,5 g 3-Chlor-2-(4-cyano-3-hydroxyphenyl)-5-trifluormethylpyridin, 1,2 g Brommethyl-cyclopropyl-keton, 1,5 g Kaliumcarbonat und 50 ml wasserfreiem Dimethylformamid wurde 40 Stunden bei 23°C gerührt. Nach Zugabe von 200 ml Wasser extrahierte man dreimal mit je 50 ml Methyl-tert.-butylether. Die vereinigten organischen Phasen wurden mit 50 ml Wasser gewaschen, dann über Natriumsulfat getrocknet und eingeengt. Der verbliebene Rückstand wurde durch Verreiben mit n-Hexan zur Kristallisation gebracht. Ausbeute: 1,6 g weißer Kristalle mit einem Schmelzpunkt von 115-116°C.

### Anwendungsbeispiele (herbizide Wirksamkeit)

Die herbizide Wirkung der substituierten 2-Phenylpyridine I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0156 oder 0,0078 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Solanum nigrum | Schwarzer Nachtschatten | black nightshade |

Bei einer Aufwandmenge von 0,0156 oder 0,0078 kg/ha a.S. zeigten die Verbindungen Nr. Ia.09 und Nr. Ih.09 im Nachauflaufverfahren eine sehr gute Wirkung gegen die o.g. Pflanzen.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°).

Die jungen Baumwollpflanzen wurden tropfnaß mit waßrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte 2-Phenylpyridine der allgemeinen Formel I 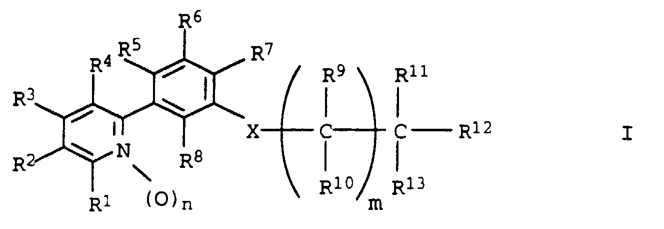 in der die Variablen folgende Bedeutung haben:
n 0 oder 1;
R¹, R³, R⁴ unabhängig voneinander
Wasserstoff, Halogen, C₁-C₄-Alkyl, C₁-C₄-Halogenalkyl, Hydroxy, C₁-C₄-Alkoxy, C₁-C₄-Halogenalkoxy, Nitro, Amino, C₁-C₄-Alkylamino, Di-(C₁-C₄-alkyl)-amino, Mercapto, C₁-C₄-Alkylthio, C₁-C₄-Halogenalkylthio, Cyano, Carboxyl, Aminocarbonyl, (C₁-C₄-Alkylamino)carbonyl oder Di-(C₁-C₄-alkyl)-aminocarbonyl;
R² Wasserstoff, Halogen, C₁-C₄-Alkyl, C₁-C₄-Halogenalkyl, Cyano, Nitro, Amino, Hydroxy, C₁-C₄-Halogenalkoxy, Mercapto, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl;
R⁵ Wasserstoff, Halogen oder Cyano;
R⁶ und R⁸ unabhängig voneinander Wasserstoff oder Halogen;
R⁷ Wasserstoff, Cyano, Nitro, Hydroxy, Trifluormethylsulfonyloxy, Halogen, C₁-C₄-Alkyl, C₁-C₄-Halogenalkyl, C₁-C₄-Alkoxy oder C₁-C₄-Halogenalkoxy;
X Sauerstoff oder Schwefel,
R⁹, R¹⁰ unabhängig voneinander Wasserstoff oder C₁-C₄-Alkyl;
m 1, 2, 3 oder 4;
R¹¹ C₁-C₆-Alkyl, C₃-C₆-Cycloalkyl, C₁-C₄-Halogenalkyl mit ein bis fünf Halogenatomen, C₂-C₄-Alkenyl, C₂-C₄-Halogenalkenyl mit ein bis drei Halogenatomen, (C₁-C₄-Alkoxy)carbonyl, (C₁-C₄-Alkoxy)carbonyl-(C₁-C₄-alkyl)-, unsubstituiertes oder ein bis dreifach substituiertes Phenyl, unsubstituiertes oder ein oder zweifach substituiertes Thienyl, Furanyl, Tetrahydrofuranyl, Pyrrolyl, Oxazolyl, Isoxazolyl, Thiazolyl, Isothiazolyl, Pyridyl oder Pyrimidyl, wobei die Substituenten jeweils ausgewählt sind aus der Gruppe bestehend aus Nitro, Cyano, Halogen, C₁-C₄-Alkyl und C₁-C₄-Alkoxy;
R¹², R¹³ unabhängig voneinander C₁-C₄-Alkoxy oder C₁-C₄-Alkyl-thio oder zusammen mit dem gemeinsamen Kohlenstoffatom, an das sie gebunden sind, die Carbonylgruppe, eine Gruppe
〉C=N―O―R¹⁴,
wobei R¹⁴ für Wasserstoff, C₁-C₄-Alkyl, C₂-C₆-Alkenyl, C₂-C₆-Halogenalkenyl mit ein bis drei Halogenatomen, (C₁-C₄-Alkoxy)carbonyl-(C₁-C₄-alkyl)- oder Benzyl steht, oder einen Heterocyclus 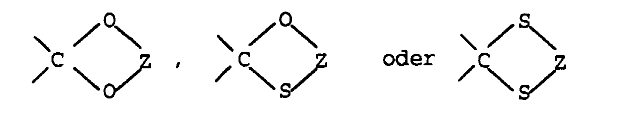 wobei Z für eine Ethylen- oder Trimethylenkette steht, bei der gewünschtenfalls ein bis vier Wasserstoffatome durch jeweils C₁-C₄-Alkyl oder (C₁-C₄-Alkoxy)carbonyl substituiert sein können,
sowie die landwirtschaftlich brauchbaren Salze der Verbindungen I, sofern diese existieren.

2. Substituierte 2-Phenylpyridine der Formel I nach Anspruch 1, wobei n für O, R¹, R³ und R⁴ unabhängig voneinander für Wasserstoff oder Halogen, R² für Halogen oder C₁-C₄-Halogenalkyl mit ein bis fünf Halogenatomen, R⁵ für Wasserstoff oder Halogen, R⁶ für Wasserstoff, R⁷ für Halogen oder Cyano, R⁸ für Wasserstoff, X für Sauerstoff, R⁹ und R¹⁰ unabhängig voneinander für Wasserstoff oder C₁-C₄-Alkyl, m für eins, R¹¹ für C₁-C₆-Alkyl oder C₃-C₆-Cycloalkyl, R¹² und R¹³ zusammen mit dem gemeinsamen Kohlenstoffatom, an das sie gebunden sind, für die Carbonylgruppe stehen.

3. Verwendung der substituierten 2-Phenylpyridine der Formel I und der landwirtschaftlich brauchbaren Salze von I, gemäß Anspruch 1, als Herbizide oder zur Desikkation und/oder Defoliation von Pflanzen.

4. Herbizides Mittel, enthaltend eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Mittel zur Desikkation und/oder Defoliation von Pflanzen, enthaltend eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

6. Verfahren zur Herstellung von herbizid wirksamen Mitteln, dadurch gekennzeichnet, daß man eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Herstellung von desikkant und/oder defoliant wirksamen Mitteln, dadurch gekennzeichnet, daß man eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

8. Verfahren zur Bekämpfung von unerwünschtem Pflanzenwuchs, dadurch gekennzeichnet, daß man eine herbizid wirksame Menge mindestens eines substituierten 2-Phenylpyridins der Formel I oder eines landwirtschaftlich brauchbaren Salzes von I, gemäß Anspruch 1, auf Pflanzen, deren Lebensraum oder auf Saatgut einwirken läßt.

9. Verfahren zur Desikkation und/oder Defoliation von Pflanzen, dadurch gekennzeichnet, daß man eine desikkant und/oder defoliant wirksame Menge mindestens eines substituierten 2-Phenylpyridins der Formel I oder eines landwirtschaftlich brauchbaren Salzes von I, gemäß Anspruch 1, auf Pflanzen einwirken läßt.

10. Verfahren nach Anspruch 9, dadurch gekennzeichnet, daß man Baumwolle behandelt.

11. Verfahren zur Herstellung von substituierten 2-Phenylpyridinen der Formel I gemäß Anspruch 1, dadurch gekennzeichnet, daß man ein 3-Pyridyl(thio)phenol der Formel III 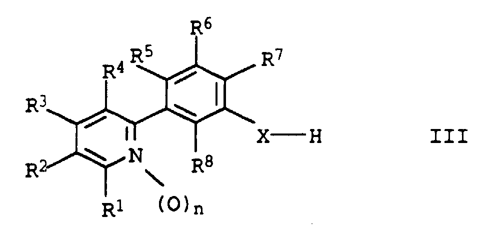 in einem inerten Lösungs- oder Verdünnungsmittel, gewünschtenfalls in Gegenwart einer Base, mit einem Alkylierungsmittel der Formel IV 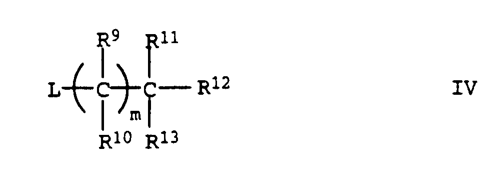 wobei L für Chlor, Brom, Jod, Methylsulfonyloxy, Trifluormethylsulfonyloxy, Phenylsulfonyloxy oder p-Tolylsulfonyloxy steht,
umsetzt.

12. Verfahren zur Herstellung von substituierten 2-Phenylpyridinen der Formel I gemäß Anspruch 1, bei denen n für 1 und X für Sauerstoff stehen, dadurch gekennzeichnet, daß man die entsprechenden substituierten 2-Phenylpyridine, bei denen n Null und X Sauerstoff bedeuten, auf an sich bekannte Weise in einem inerten Lösungs- oder Verdünnungsmittel oxidiert.

## Claims

1. A substituted 2-phenylpyridine of the general formula I 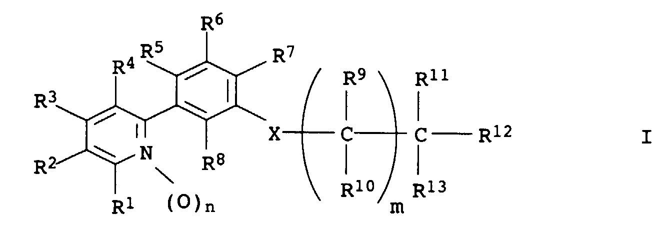 where the variables have the following meanings:
n is 0 or 1;
R¹, R³ and R⁴ independently of one another are
hydrogen, halogen, C₁-C₄-alkyl, C₁-C₄-haloalkyl, hydroxyl, C₁-C₄-alkoxy, C₁-C₄-haloalkoxy, nitro, amino, C₁-C₄-alkyl-amino, di-(C₁-C₄-alkyl)-amino, mercapto, C₁-C₄-alkylthio, C₁-C₄-haloalkylthio, cyano, carboxyl, aminocarbonyl, (C₁-C₄-alkylamino)carbonyl or di-(C₁-C₄-alkyl)-amino-carbonyl;
R² is hydrogen, halogen, C₁-C₄-alkyl, C₁-C₄-haloalkyl, cyano, nitro, amino, hydroxyl, C₁-C₄-haloalkoxy, mercapto, C₁-C₄-alkylthio, C₁-C₄-haloalkylthio, C₁-C₄-alkylsulfinyl, C₁-C₄-haloalkylsulfinyl, C₁-C₄-alkylsulfonyl or C₁-C₄-haloalkylsulfonyl;
R⁵ is hydrogen, halogen or cyano;
R⁶ and R⁸ independently of one another are hydrogen or halogen;
R⁷ is hydrogen, cyano, nitro, hydroxyl, trifluoromethylsulfonyloxy, halogen, C₁-C₄-alkyl, C₁-C₄-haloalkyl, C₁-C₄-alkoxy or C₁-C₄-haloalkoxy;
X is oxygen or sulfur,
R⁹ and R¹⁰ independently of one another are hydrogen or C₁-C₄-alkyl;
m is 1, 2, 3 or 4;
R¹¹ is C₁-C₆-alkyl, C₃-C₆-cycloalkyl, C₁-C₄-haloalkyl having one to five halogen atoms, C₂-C₄-alkenyl, C₂-C₄-haloalkenyl having one to three halogen atoms, (C₁-C₄-alkoxy)carbonyl, (C₁-C₄-alkoxy)carbonyl-(C₁-C₄-alkyl), unsubstituted or mono- to trisubstituted phenyl, unsubstituted or mono- or disubstituted thienyl, furanyl, tetrahydrofuranyl, pyrrolyl, oxazolyl, isoxazolyl, thiazolyl, isothiazolyl, pyridyl or pyrimidyl, where the substituents selected in each case are from the group consisting of nitro, cyano, halogen, C₁-C₄-alkyl and C₁-C₄-alkoxy;
R¹² and R¹³ independently of one another are C₁-C₄-alkoxy or C₁-C₄-alkylthio or, together with the common carbon atom to which they are bonded, are the carbonyl group, a group
〉C=N―O―R¹⁴,
where R¹⁴ is hydrogen, C₁-C₄-alkyl, C₂-C₆-alkenyl, C₂-C₆-haloalkenyl having one to three halogen atoms, (C₁-C₄-alkoxy)carbonyl-(C₁-C₄-alkyl) or benzyl, or a heterocycle 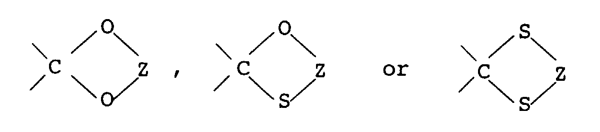 where Z is an ethylene or trimethylene chain in which, if desired, one to four hydrogen atoms can be substituted by C₁-C₄-alkyl or (C₁-C₄-alkoxy)carbonyl in each case,
or the agriculturally utilizable salts of the compounds I, if these exist.

2. A substituted 2-phenylpyridine of the formula I as claimed in claim 1, where n is 0, R¹, R³ and R⁴ independently of one another are hydrogen or halogen, R² is halogen or C₁-C₄-haloalkyl having one to five halogen atoms, R⁵ is hydrogen or halogen, R⁶ is hydrogen, R⁷ is halogen or cyano, R⁸ is hydrogen, X is oxygen, R⁹ and R¹⁰ independently of one another are hydrogen or C₁-C₄-alkyl, m is one, R¹¹ is C₁-C₆-alkyl or C₃-C₆-cycloalkyl, R¹² and R¹³, together with the common carbon atom to which they are bonded, are the carbonyl group.

3. The use of the substituted 2-phenylpyridines of the formula I and of the agriculturally utilizable salts of I, as claimed in claim 1, as herbicides or for the desiccation and/or defoliation of plants.

4. A herbicidal composition containing a herbicidally active amount of at least one substituted 2-phenylpyridine of the formula I or of an agriculturally utilizable salt of I, as claimed in claim 1, and at least one inert liquid and/or solid carrier and also, if desired, at least one surface-active substance.

5. A composition for the desiccation and/or defoliation of plants, containing an amount of at least one substituted 2-phenylpyridine of the formula I or of an agriculturally utilizable salt of I, as claimed in claim 1, which has desiccant and/or defoliant activity, and at least one inert liquid and/or solid carrier and also, if desired, at least one surface-active substance.

6. A process for preparing herbicidally active compositions, which comprises mixing a herbicidally active amount of at least one substituted 2-phenylpyridine of the formula I or of an agriculturally utilizable salt of I, as claimed in claim 1, and at least one inert liquid and/or solid carrier and also, if desired, at least one surface-active substance.

7. A process for preparing compositions having desiccant and/or defoliant activity, which comprises mixing an amount of at least one substituted 2-phenylpyridine of the formula I or of an agriculturally utilizable salt of I, as claimed in claim 1, having desiccant and/or defoliant activity, and at least one inert liquid and/or solid carrier and also, if desired, at least one surface-active substance.

8. A method of controlling undesired vegetation, which comprises allowing a herbicidally active amount of at least one substituted 2-phenylpyridine of the formula I or of an agriculturally utilizable salt of I, as claimed in claim 1, to act on plants, their habitat or on seed.

9. A method for the desiccation and/or defoliation of plants, which comprises allowing an amount of at least one substituted 2-phenylpyridine of the formula I or of an agriculturally utilizable salt of I, as claimed in claim 1, having desiccant and/or defoliant activity, to act on plants.

10. A method as claimed in claim 9, wherein cotton is treated.

11. A process for preparing substituted 2-phenylpyridines of the formula I as claimed in claim 1, which comprises reacting a 3-pyridyl(thio)phenol of the formula III 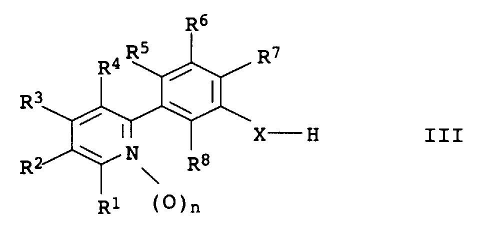 in an inert solvent or diluent, if desired in the presence of a base, with an alkylating agent of the formula IV 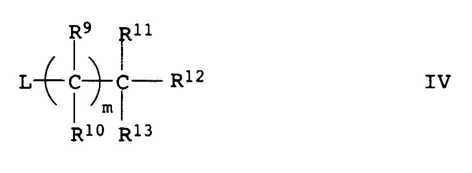 where L is chlorine, bromine, iodine, methylsulfonyloxy, trifluoromethylsulfonyloxy, phenylsulfonyloxy or p-tolylsulfonyloxy.

12. A process for preparing substituted 2-phenylpyridines of the formula I as claimed in claim 1, where n is 1 and X is oxygen, which comprises oxidizing the corresponding substituted 2-phenylpyridines, where n is zero and X is oxygen, in a manner known per se in an inert solvent or diluent.

## Revendications

1. 2-phénylpyridines substituées de formule générale I 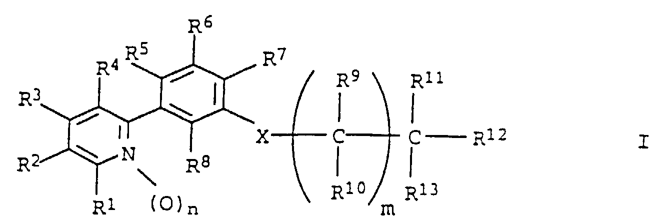 dans laquelle les symboles ont les significations suivantes :
n est égal à 0 ou 1 ;
R¹, R³ et R⁴ représentent chacun, indépendamment les uns des autres, l'hydrogène, un halogène, un groupe alkyle en C1-C4, halogénoalkyle en C1-C4, hydroxy, alcoxy en C1-C4, halogénoalcoxy en C1-C4, nitro, amino, alkylamino en C1-C4, di-(alkyle en C1-C4)amino, mercapto, alkylthio en C1-C4, halogénoalkylthio en C1-C4, cyano, carboxyle, aminocarbonyle, (alkylamino en C1-C4)carbonyle ou di-(alkyle en C1-C4)aminocarbonyle ;
R² représente l'hydrogène, un halogène, un groupe alkyle en C1-C4, halogénoalkyle en C1-C4, cyano, nitro, amino, hydroxy, halogénoalcoxy en C1-C4, mercapto, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4 ;
R⁵ représente l'hydrogène, un halogène ou un groupe cyano ;
R⁶ et R⁸ représentent chacun, indépendamment l'un de l'autre, l'hydrogène ou un halogène ;
R⁷ représente l'hydrogène, un groupe cyano, nitro, hydroxy, trifluorométhylsulfonyloxy, un halogène, un groupe alkyle en C1-C4, halogénoalkyle en C1-C4, alcoxy en C1-C4 ou halogénoalcoxy en C1-C4 ;
X représente l'oxygène ou le soufre,
R⁹, R¹⁰ représentent chacun, indépendamment l'un de l'autre, l'hydrogène ou un groupe alkyle en C1-C4 ;
m est égal à 1, 2, 3 ou 4 ;
R¹¹ représente un groupe alkyle en C1-C6, cycloalkyle en C3-C6, halogénoalkyle en C1-C4 contenant un à cinq atomes d'halogènes, alcényle en C2-C4, halogénoalcényle en C2-C4 contenant un à trois atomes d'halogènes, (alcoxy en C1-C4)carbonyle, (alcoxy en C1-C4)carbonyl-alkyle en C1-C4, phényle non substitué ou portant un à trois substituants, thiényle, furannyle, tétrahydrofurannyle, pyrrolyle, oxazolyle, isoxazolyle, thiazolyle, isothiazolyle, pyridyle ou pyrimidyle non substitué ou portant un ou deux substituants, les substituants en question étant choisis parmi les groupes nitro, cyano, les halogènes, les groupes alkyle en C1-C4 et alcoxy en C1-C4 ;
R¹² et R¹³ représentent chacun, indépendamment l'un de l'autre, un groupe alcoxy en C1-C4 ou alkylthio en C1-C4 ou bien, ensemble et avec l'atome de carbone auquel ils sont reliés, un groupe carbonyle, un groupe
〉C=N―O―R¹⁴,
dans lequel R¹⁴ représente l'hydrogène, un groupe alkyle en C1-C4, alcényle en C2-C6, halogénoalcényle en C2-C6 contenant un à trois atomes d'halogènes, (alcoxy en C1-C4)carbonyl-alkyle en C1-C4 ou benzyle, ou bien un hétérocycle 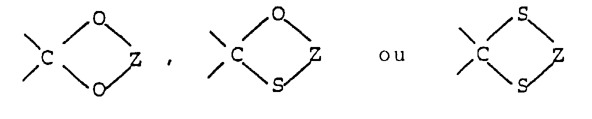 dans lequel Z représente une chaîne éthylène ou triméthylène dans laquelle, si on le désire, on peut remplacer un à quatre atomes d'hydrogène par des groupes alkyle en C1-C4 ou (alcoxy en C1-C4)carbonyle,
ainsi que les sels des composés I convenant pour les applications agricoles, pour autant qu'ils existent.

2. 2-phénylpyridines substituées de formule I selon la revendication 1, dans laquelle n est égal à 0, R¹, R³ et R⁴ représentent chacun, indépendamment les uns des autres, l'hydrogène ou un halogène, R² représente un halogène ou un groupe halogénoalkyle en C1-C4 contenant un à cinq atomes d'halogènes, R⁵ représente l'hydrogène ou un halogène, R⁶ représente l'hydrogène, R⁷ un halogène ou un groupe cyano, R⁸ l'hydrogène, X l'oxygène, R⁹ et R¹⁰ représentent chacun, indépendamment l'un de l'autre, l'hydrogène ou un groupe alkyle en C1-C4, m est égal à 1, R¹¹ représente un groupe alkyle en C1-C6 ou cycloalkyle en C3-C6, R¹² et R¹³ forment ensemble et avec l'atome de carbone auquel ils sont reliés le groupe carbonyle.

3. Utilisation des 2-phénylpyridines substituées de formule I et des sels de I convenant pour les applications agricoles, selon la revendication 1, en tant que produits herbicides pour la dessication et/ou la défoliation des végétaux.

4. Produit herbicide contenant une quantité herbicide efficace d'au moins une 2-phénylpyridine substituée de formule I ou d'un sel de I acceptable pour les applications agricoles, selon la revendication 1, et au moins un véhicule liquide et/ou solide inerte et, le cas échéant, au moins une substance tensio-active.

5. Produit pour la dessication et/ou la défoliation des végétaux, contenant une quantité efficace d'au moins une 2-phénylpyridine substituée de formule I ou d'un sel de I convenant pour les applications agricoles, selon la revendication 1, et au moins un véhicule liquide et/ou solide inerte et, si on le désire, au moins une substance tensio-active.

6. Procédé pour la préparation de produits herbicides, caractérisé par le fait que l'on mélange une quantité herbicide efficace d'au moins une 2-phénylpyridine substituée de formule I ou d'un sel de I convenant pour les applications agricoles, selon la revendication 1, et au moins un véhicule liquide et/ou solide inerte, ainsi que, le cas échéant, au moins une substance tensio-active.

7. Procédé pour la préparation de produits dessiccants et/ou défoliants, caractérisé par le fait que l'on mélange une quantité efficace d'au moins une 2-phénylpyridine substituée de formule I ou d'un sel de I convenant pour les applications agricoles, selon la revendication 1, et au moins un véhicule liquide et/ou solide inerte ainsi que, le cas échéant, au moins une substance tensio-active.

8. Procédé pour combattre les croissances de végétaux indésirables, caractérisé par le fait que l'on fait agir sur les végétaux, leur habitat ou leurs semences, une quantité herbicide efficace d'au moins une 2-phénylpyridine substituée de formule I ou d'un sel de I convenant pour les applications agricoles, selon la revendication 1.

9. Procédé pour la dessiccation et/ou la défoliation de végétaux, caractérisé par le fait que l'on fait agir sur les végétaux une quantité efficace d'au moins une 2-phénylpyridine substituée de formule I ou d'un sel de I convenant pour les applications agricoles, selon la revendication 1.

10. Procédé selon la revendication 9, caractérisé par le fait que l'on traite le coton.

11. Procédé pour la préparation des 2-phénylpyridines substituées de formule I selon la revendication 1, caractérisé par le fait que l'on fait réagir un 3-pyridyl(thio)phénol de formule III 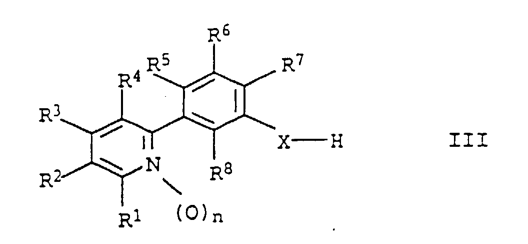 dans un solvant ou diluant inerte et le cas échéant en présence d'une base, avec un agent alkylant de formule IV 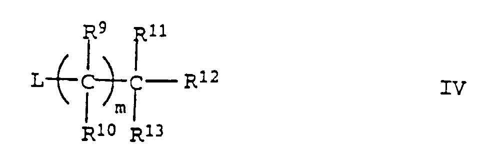 dans laquelle L représente le chlore, le brome, l'iode, un groupe méthylsulfonyloxy, trifluorométhylsulfonyloxy, phénylsulfonyloxy ou p-tolylsulfonyloxy.

12. Procédé de préparation des 2-phénylpyridines substituées de formule I selon la revendication 1 dans laquelle n est égal à 1 et X représente l'oxygène, caractérisé par le fait que l'on oxyde de manière connue en soi, dans un solvant ou diluant inerte, les 2-phénylpyridines substituées correspondantes pour lesquelles n est égal à 0 et X représente l'oxygène.
